# EUROPEAN PATENT APPLICATION

(11) **EP 1 666 035 A1**
(43) Date of publication of application: **07.06.2006**
(21) Application number: 04787963.0
(22) Date of filing: 22.09.2004
(51) Int. Cl.: A61K 31/343, C07D 307/79, A61P 1/16, A61P 35/00, A61P 31/12, A61P 31/04

(54) **MEDICINAL COMPOSITION FOR TREATMENT FOR FATTY LIVER OR LIVER DISEASE**

(30) Priority: 26.09.2003 JP 2003335639
(71) Applicant: CHUGAI SEIYAKU KABUSHIKI KAISHA, Tokyo, 115-8543 (JP)
(72) Inventor: CYNSHI, Osamu, Chugai Seiyaku Kabushiki Kaisha, Gotenba-shi, Shizuoka 412-8513 (JP); KOMORI, Toshihiko, c/o Chugai Seiyaku K. K., Tokyo 104-8301 (JP); KAISE, Hiroshi, Chugai Seiyaku Kabushiki Kaisha, Tokyo 115-8543 (JP); TAKEDA, Minako, Chugai Seiyaku Kabushiki Kaisha, Gotenba-shi, Shizuoka 412-8513 (JP); KAWABE, Yoshiki, Chugai Seiyaku Kabushiki Kaisha, Gotenba-shi, Shizuoka 412-8513 (JP)
(74) Representative: Vossius & Partner
(86) International application number: PCT/JP2004/013780
(87) International publication number: WO 2005/030198

(57) **Abstract**

A pharmaceutical composition, and a method using the composition, for prevention and/or treatment of fatty liver or hepatic disease, the composition comprising, as an active ingredient, a compound of the formula (1): 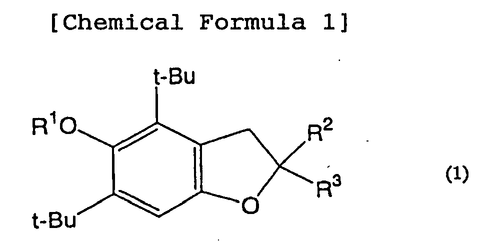 where
R¹ is a hydrogen atom, an acyl group, or an arylalkoxycarbonyl group; and
R² and R³ are each independently a substituted or unsubstituted alkyl group, a substituted or unsubstituted alkenyl group, or a substituted or unsubstituted alkynyl group, or R² and R³ may combine to form a cycloalkyl group.

## Description

### TECHNICAL FIELD

This invention relates to a pharmaceutical composition for prevention and/or treatment of fatty liver or hepatic disease, and a method for prevention and/or treatment of fatty liver or hepatic disease.

### BACKGROUND ART

The liver is an important organ which performs the metabolism/detoxication of drugs and poisons by its metabolic activities. It is also important as a regulatory organ for maintaining substances necessary for maintenance of organisms, such as cholesterol, at constant concentrations in the blood.

Metabolism in the liver is performed by the endoplasmic reticula of the liver cells. These endoplasmic reticula contain the most lipids among the organelles of the hepatocytes. Thus, the frequency of hyperoxidation of lipids occurring there is considered to be higher than in other parts of the liver. Lipid peroxides produced in these endoplasmic reticula were reported to be clinically important causes of hepatic disorders (M. Uchiyama, M. Matsuo and M. Sagai, Lipid peroxides and organism, p. 293-295, 1985, Japan Scientific Societies Press, Tokyo).

In recent years, cells constituting the walls of the hepatic sinusoids, such as Kupffer cells which are macrophages residing in the liver, have been reported to play important roles in the etiology and pathogenesis of hepatic diseases (M. Inoue, Active oxygen and pathogenesis - To bedside for fear of disease, p. 379-390, 1992, Japan Scientific Societies Press, Tokyo). For example, free radicals generated upon activation of Kupffer cells are assumed to cause cytotoxicity, leading to the occurrence or progression of hepatic disease.

Furthermore, free radicals generated in association with alcohol metabolism were reported to play an important role in the progression of alcoholic fatty liver due to excessive ingestion of alcohol (Par A, Roth E, Rumi G, Kovacs Z, Nemes J, Mozsik G. Oxidative stress and antioxidant defense in alcoholic liver disease and chronic hepatitis C. Orv Hetil 2000 141:1559-655). The generated free radicals are presumed to cause injury to liver cells.

Based on these reports, oxidative stress, including lipid hyperoxidation by radicals, is presumed to be involved in the onset or progression of various hepatic diseases, and to cause damage to cells. Thus, substances capable of diminishing oxidative stress, namely, antioxidants, have been expected to be effective for the prevention or treatment of various hepatic diseases. Thus, many clinical studies have been conducted using antioxidants.

Antioxidants lowered various indicators showing buildup of oxidative stress in hepatic diseases (Loguercio C, Federico A. Oxidative stress in viral and alcoholic hepatitis. Free Radic Biol Med 2003 34:1-10; Zima T, Fialova L, Mestek O, Janeva M, Crkovka J, Malbohan I, Stipek S, Mikulikova L, Popov P. Oxidative stress, metabolism of ethanol and alcohol-related diseases. J Biomed Sci 2001, 8:59-70; Prince MI, Mitchison HC, Ashley D, Burke DA, Edwards N, Bramble MG, James OF, Jones DE. Oral antioxidant supplementation for fatigue associated with primary biliary cirrhosis: results of a multicentre, randomized, placebo-controlled, cross-over trial. Aliment Pharmacol Ther. 2003 17:137-43). However, the antioxidants merely suppressed the buildup of oxidative stress, and did not ameliorate hepatic diseases per se. This was true of antioxidant vitamins which have the ability to protect liver cells from injury due to lipid hyperoxidation caused by radicals (Prince MI, Mitchison HC, Ashley D, Burke DA, Edwards N, Bramble MG, James OF, Jones DE. Oral antioxidant supplementation for fatigue associated with primary biliary cirrhosis: results of a multicentre, randomized, placebo-controlled, cross-over trial. Aliment Pharmacol Ther. 2003 17:137-43; Houglum K, Venkataramani A, Lyche K, Chojkier M. A pilot study of the effects of d-alpha-tocopherol on hepatic stellate cell activation in chronic hepatitis C. Gastroenterology. 1997 113:1069-73).

The effects of antioxidant vitamins on hepatic diseases associated with nonalcoholic fatty liver have also been investigated, but no conclusions have been obtained (Lavine JE. Vitamin E treatment of nonalcoholic steatohepatitis in children: a pilot study. J Pediatr. 2000 136:734-8; Hasegawa T, Yoneda M, Nakamura K, Makino I, Terano A. Plasma transforming growth factor-betal level and efficacy of alpha-tocopherol in patients with non-alcoholic steatohepatitis: a pilot study. Aliment Pharmacol Ther. 2001 15:1667-72). Nonalcoholic fatty liver is fatty liver which has increased in recent years with increases in diabetes and obesity, and it has been pointed out that no effective therapeutic agents have been present for hepatic diseases due to this pathologic state (Angulo P. Nonalcoholic fatty liver disease. N Engl J Med. 2002 346:1221-31).

These results of the studies suggest that mere protection of liver cells from injury due to radicals is not enough for the prevention and treatment of hepatic diseases.

The inventors reported that 4,6-di-t-butyl-5-hydroxy-2,2-di-n-pentyl-2,3-dihydrobenzofuran (hereinafter referred to as BO-653) was effective against arteriosclerosis, because it suppressed the oxidation of low density lipoprotein (LDL) (Cynshi O, Kawabe Y, Suzuki T, Takashima Y, Kaise H, Nakamura M, Ohba Y, Kato Y, Tamura K, Hayasaka A, Higashida A, Sakaguchi H, Takeya M, Takahashi K, Inoue K, Noguchi N, Niki E, Kodama T. Antiatherogenic effects of the antioxidant BO-653 in three different animal models. Proc Natl Acad Sci USA. 1998 95:10123-8). The inventors also disclosed that BO-653 and its analogous compounds have an antioxidant action and a lipid hyperoxidation suppressing action, and thus they are useful as therapeutic agents for arteriosclerosis and useful for treatment of ischemic organ disorders such as myocardial infarction and cerebral apoplexy (JP 6-206842A/1994; US 5,574,178), they are useful as agents for suppressing tunica intima hypertrophy, and as agents for suppressing restenosis after percutaneous transluminal coronary angioplasty (PTCA) (JP 9-188619A/1997; US 6,103,753), they are useful as therapeutic or prophylactic agents for renal diseases, and useful as agents for organ preservation (JP 10-72458A/1998; US 6,133,279), they are useful as prophylactic or therapeutic agents for atherosclerosis or xanthoma (JP 11-21238A/1999; US 6,156,793; US 6,417,225), and they are useful as drugs for decreasing the incidence of, or alleviating symptoms of, acute coronary syndrome (WO03/018001).

However, it has been neither disclosed nor suggested that BO-653 and its analogous compounds are useful as agents for prophylaxis and/or treatment of fatty liver or hepatic disease.
Patent document 1: JP 6-206842A/1994
Patent document 2: US 5,574,178
Patent document 3: JP 9-188619A/1997
Patent document 4: US 6,103,753
Patent document 5: JP 10-72458A/1998
Patent document 6: US 6,133,279
Patent document 7: JP 11-21238A/1999
Patent document 8: US 6,156,793
Patent document 9: US 6,417,225
Patent document 10: WO03/018001
Non-patent document 1: M. Uchiyama, M. Matsuo and M. Sagai, Lipid peroxides and organism, p. 293-295, 1985, Japan Scientific Societies Press, Tokyo
Non-patent document 2: M. Inoue, Active oxygen and pathogenesis - To bedside for fear of disease, p. 379-390, 1992, Japan Scientific Societies Press, Tokyo
Non-patent document 3: Par A, Roth E, Rumi G, Kovacs Z, Nemes J, Mozsik G., Oxidative stress and antioxidant defense in alcoholic liver disease and chronic hepatitis C. Orv Hetil 2000 141:1559-655
Non-patent document 4: Loguercio C, Federico A. Oxidative stress in viral and alcoholic hepatitis. Free Radic Biol Med 2003 34:1-10
Non-patent document 5: Zima T, Fialova L, Mestek O, Janeva M, Crkovka J, Malbohan I, Stipek S, Mikulikova L, Popov P. Oxidative stress, metabolism of ethanol and alcohol-related disease. J Biomed Sci 2001, 8:59-70
Non-patent document 6: Prince MI, Mitchison HC, Ashley D, Burke DA, Edwards N, Bramble MG, James OF, Jones DE. Oral antioxidant supplementation for fatigue associated with primary biliary cirrhosis: results of a multicentre, randomized, placebo-controlled, cross-over trial. Aliment Pharmacol Ther. 2003 17:137-43
Non-patent document 7: Houglum K, Venkataramani A, Lyche K, Chojkier M. A pilot study of the effects of d-alpha-tocopherol on hepatic stellate cell activation in chronic hepatitis C. Gastroenterology. 1997 113:1069-73
Non-patent document 8: Lavine JE. Vitamin E treatment of nonalcoholic steatohepatitis in children: a pilot study. J Pediatr. 2000 136:734-8
Non-patent document 9: Hasegawa T, Yoneda M, Nakamura K, Makino I, Terano A. Plasma transforming growth factor-betal level and efficacy of alpha-tocopherol in patients with non-alcoholic steatohepatitis: a pilot study. Aliment Pharmacol Ther. 2001 15:1667-72
Non-patent document 10: Angulo P. Nonalcoholic fatty liver disease. N Engl J Med. 2002 346:1221-31
Non-patent document 11: Cynshi O, Kawabe Y, Suzuki T, Takashima Y, Kaise H, Nakamura M, Ohba Y, Kato Y, Tamura K, Hayasaka A, Higashida A, Sakaguchi H, Takeya M, Takahashi K, Inoue K, Noguchi N, Niki E, Kodama T. Antiatherogenic effects of the antioxidant BO-653 in three different animal models. Proc Natl Acad Sci USA. 1998 95:10123-8

### DISCLOSURE OF THE INVENTION

### PROBLEMS TO BE SOLVED BY THE INVENTION

It is an object of the present invention to provide a pharmaceutical composition for prevention and/or treatment of fatty liver or hepatic disease, especially, fatty liver or hepatic disease associated with fatty liver, and a method for prevention and/or treatment of fatty liver or hepatic disease.

### MEANS FOR SOLVING THE PROBLEMS

The inventors diligently conducted studies, and have found that in mice which had fatty liver because of high fat diet feeding, and developed hepatic diseases associated with the fatty liver, certain 4,6-di-t-butyldihydrobenzofuran derivatives reduce the amount of the enzyme aspartate aminotransferase (hereinafter referred to as AST) leaking from liver cells into the blood, and also suppress hepatomegaly.

The present invention provides a pharmaceutical composition for prevention and/or treatment of fatty liver or hepatic disease, which comprises, as an active ingredient, a compound of the formula (1):

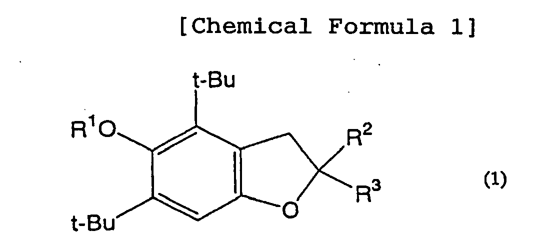

where
R¹ is a hydrogen atom, an acyl group, or an arylalkoxycarbonyl group; and
R² and R³ are each independently a substituted or unsubstituted alkyl group, a substituted or unsubstituted alkenyl group, or a substituted or unsubstituted alkynyl group, or R² and R³ may combine to form a cycloalkyl group.

In an embodiment of the present invention, leakage of hepatic enzymes due to hepatopathy is suppressed, and progression of hepatic diseases is inhibited accordingly.

In a preferred embodiment of the present invention, the hepatic disease is a hepatic disease associated with fatty liver and, more preferably, a hepatic disease associated with nonalcoholic fatty liver.

In another embodiment of the present invention, the hepatic disease is hepatic function disorder of a bacterial origin or due to a chemical, and includes chronic and acute hepatitis. In still another embodiment, the hepatic disease is viral hepatitis or hepatic disease, or liver cancer.

The present invention also provides use of the compound of the formula (1) in the production of a pharmaceutical composition for prevention and/or treatment of fatty liver or hepatic disease.

Furthermore, the present invention provides a method for prevention and/or treatment of fatty liver or hepatic disease, which comprises administering the compound of the formula (1) to a patient in need of such prevention and/or treatment.

In the compound of the formula (1) according to the present invention, R¹ is a hydrogen atom, an acyl group, or an arylalkoxycarbonyl group. The preferred acyl group is an acyl group having 1 to 10 carbon atoms, and its examples include formyl, acetyl, propionyl, and benzoyl groups. The preferred arylalkyloxycarbonyl group is an arylalkyloxycarbonyl group having 7 to 11 carbon atoms, and its examples include benzyloxycarbonyl and naphthylmethyloxycarbonyl groups.

The preferred examples of R¹ are a hydrogen atom and an acyl group. A hydrogen atom and an acetyl group are more preferred, and a hydrogen atom is particularly preferred.

In the compound of the formula (1), R² and R³ are each independently a substituted or unsubstituted alkyl group, a substituted or unsubstituted alkenyl group, or a substituted or unsubstituted alkynyl group.

The preferred alkyl group is a straight chain or branched chain alkyl group having 1 to 20 carbon atoms, and its examples include methyl, ethyl, n-propyl, isopropyl, n-butyl, isobutyl, s-butyl, t-butyl, n-pentyl, isopentyl, sec-pentyl, t-pentyl, neopentyl, n-hexyl, isohexyl, ethylbutyl, n-heptyl, isoheptyl, ethylpentyl, n-octyl, ethylhexyl, propylpentyl, nonyl, decyl, pentadecyl, and stearyl groups. More preferred is a straight chain or branched chain alkyl group having 1 to 10 carbon atoms. Particularly preferred is a straight chain alkyl group having 3 to 8 carbon atoms.

The preferred alkenyl group is a straight chain or branched chain alkyl group having 2 to 20 carbon atoms. Its examples include ethenyl, propenyl, isopropenyl, butenyl, isobutenyl, pentenyl, isopentenyl, hexenyl, isohexenyl, ethylbutenyl, heptenyl, isoheptenyl, ethylpentenyl, octenyl, nonenyl, decenyl, and pentadecenyl groups. More preferred is a straight chain or branched chain alkenyl group having 2 to 10 carbon atoms. Particularly preferred is a straight chain alkenyl group having 3 to 8 carbon atoms.

The preferred alkynyl group is a straight chain or branched chain alkynyl group having 2 to 20, preferably 2 to 10, carbon atoms. Particularly preferred is a straight chain alkynyl group having 3 to 8 carbon atoms. Its examples include alkynyl groups corresponding to the examples named concerning the alkenyl group.

R² and R³ may combine to form a cycloalkyl group having 5 to 10 carbon atoms. Preferred examples of the cycloalkyl group include cyclopentyl, cyclohexyl, cycloheptyl, cyclooctyl, cyclononyl and cyclodecyl.

If R² and R³ are each an alkyl group, an alkenyl group, or an alkynyl group, examples of the substituent that they can have include halogen, lower alkoxy, hydroxy, amino, nitro and trifluoromethyl groups.

The preferred R² and R³ are each a straight chain unsubstituted alkyl group having 3 to 8 carbon atoms. It is particularly preferred that both of R² and R³ are n-butyl groups, n-pentyl groups, n-hexyl groups, or n-heptyl groups. It is the most preferred that both of R² and R³ are n-pentyl groups.

The preferred compounds of the formula (1) are as follows:
4,6-Di-t-butyl-5-hydroxy-2,2-dimethyl-2,3-dihydrobenzofuran;
4,6-Di-t-butyl-5-hydroxy-2,2-diethyl-2,3-dihydrobenzofuran;
4,6-Di-t-butyl-5-hydroxy-2,2-di-n-propyl-2,3-dihydrobenzofuran;
4,6-Di-t-butyl-5-hydroxy-2,2-di-isopropyl-2,3-dihydrobenzofuran;
4,6-Di-t-butyl-5-hydroxy-2,2-di-n-butyl-2,3-dihydrobenzofuran;
4,6-Di-t-butyl-5-hydroxy-2,2-di-s-butyl-2,3-dihydrobenzofuran;
4,6-Di-t-butyl-5-hydroxy-2,2-di-t-butyl-2,3-dihydrobenzofuran;
4,6-Di-t-butyl-5-hydroxy-2,2-di-n-pentyl-2,3-dihydrobenzofuran;
4,6-Di-t-butyl-5-hydroxy-2,2-di-t-pentyl-2,3-dihydrobenzofuran;
4,6-Di-t-butyl-5-hydroxy-2,2-di-isopentyl-2,3-dihydrobenzofuran;
4,6-Di-t-butyl-5-hydroxy-2,2-di-neopentyl-2,3-dihydrobenzofuran;
4,6-Di-t-butyl-5-hydroxy-2,2-di-n-hexyl-2,3-dihydrobenzofuran;
4,6-Di-t-butyl-5-hydroxy-2,2-di-n-heptyl-2,3-dihydrobenzofuran;
4,6-Di-t-butyl-5-hydroxy-2,2-di-n-octyl-2,3-dihydrobenzofuran;
4,6-Di-t-butyl-5-hydroxy-2,2-di-n-nonyl-2,3-dihydrobenzofuran; and
4,6-Di-t-butyl-5-hydroxy-2,2-di-n-decyl-2,3-dihydrobenzofuran.

The particularly preferred compounds of the formula (1) are 4,6-di-t-butyl-5-hydroxy-2,2-di-n-butyl-2,3-dihydrobenzofuran, 4,6-di-t-butyl-5-hydroxy-2,2-di-n-pentyl-2,3-dihydrobenzofuran, 4,6-di-t-butyl-5-hydroxy-2,2-di-n-hexyl-2,3-dihydrobenzofuran, and 4,6-di-t-butyl-5-hydroxy-2,2-di-n-heptyl-2,3-dihydrobenzofuran. The most preferred compound is 4,6-di-t-butyl-5-hydroxy-2,2-di-n-pentyl-2,3-dihydrobenzofuran.

The compounds of the formula (1) used in the present invention can be synthesized, for example, by the methods described in JP 6-206842A/1994, US 5,574,178 corresponding thereto, WO02/06263, EP1304328A corresponding thereto, and Tamura K, Kato Y, Ishikawa A, Kato Y, Himori M, Yoshida M, Takashima Y, Suzuki T, Kawabe Y, Cynshi O, Kodama T, Niki E, Shimizu M. Design and synthesis of 4,6-di-tert-butyl-2,3-dihydro-5-benzofuranols as a novel series of antiatherogenic antioxidants. J Med Chem. 2003 46:3083-93.

The hepatic disease herein refers to a disease which involves the leakage of hepatic enzymes, such as AST, by damage caused to cells constituting the liver. The hepatic disease includes, concretely, hepatic diseases associated with fatty liver, nonalcoholic fatty liver diseases, bacterial or chemical-induced hepatic function disorder, chronic or acute hepatitis, viral hepatitis, hepatic cirrhosis, liver cancer, and fatty liver. Enzyme leakage from the liver cells means that the liver cells are injured by various causes, such as accelerated hepatic metabolism due to activation of Kupffer cells or hyperlipidemia, and infection, to leak intracellular enzymes into the blood. This hepatic enzyme leakage increases with the onset and progression of hepatic disease. The leakage suppressing action of the compounds of the present invention on hepatic enzymes is considered to be the outcome of suppression of damage to the liver cells.

The pharmaceutical composition of the present invention can be made into various dosage forms by blending the compounds of the formula (1), which are the active ingredients, with physiologically acceptable solid or liquid carriers for pharmaceutical manufacturing in accordance with the routes of administration. The routes of administration include oral administration, parenteral administration such as intravenous injection, sustained release administration by sustained release preparations, and topical administration by catheters for topical administration, etc. The carriers for pharmaceutical manufacturing include vehicles, binders, disintegrants, tablet lubricants, coating agents, solution adjuvants, emulsifying agents, suspending agents, stabilizers, fats and oils, and solvents. The dosage forms include tablets, granules, pills, capsules, aqueous solutions or dispersions, syrups, suspensions, emulsions, and injections. Concretely, the compounds of the formula (1) can be pharmaceutically manufactured as seam soft capsules (WO00/50029, corresponding EP1172104A, etc.), seamless soft capsules (WO02/13819, corresponding EP1314424A, etc.), as have been reported so far.

The dose of the compound of the formula (1) according to the present invention varies according to the age of the patient, the severity of symptoms, the route of administration, etc. For example, the daily dose for adults is 1 to 1,000 mg, preferably 10 to 200 mg. This dose may be administered as a single dose or as several divided doses.

### EFFECTS OF THE INVENTION

According to the present invention, there is provided a pharmaceutical composition which can suppress the leakage of enzymes from the liver cells, and can also suppress hepatomegaly in fatty liver models, and thus, is useful for prevention and/or treatment of hepatic disease, especially, hepatic disease associated with fatty liver.

### Examples

### Test Example 1: Action of BO-653 on excess apoAI expressing mice fed with high fat diet

The therapeutic activity of BO-653 against fatty liver and associated hepatic disease was investigated using excess apoAI expressing mice which were fed with a high fat diet to induce fatty liver and further develop hepatic diseases.

ApoAI, i.e., apolipoprotein AI, is an apolipoprotein constituting high density lipoproteins mainly responsible for blood lipid metabolism in rodents including mice. In mice expressing excess apoAI, blood cholesterol levels are originally high. Thus, even if they are fed with a high fat diet for causing the onset of fatty liver, increases in blood cholesterol are relatively small. Such mice are advantageous in that the influence of changes in blood cholesterol on the results of the test need not be considered in evaluating the therapeutic activity of BO-653 against fatty liver and associated hepatic disease.

Mice expressing excess apoAI (female, 10 to 11 weeks of age, purchased from Jackson Laboratories) were divided into 4 groups of N-animals. The animals of the respective groups were fed with a normal diet, a high fat diet, a 0.6% BO-653-containing high fat diet (hereinafter referred to as B+ high fat diet), and a 0.5% probucol-containing high fat diet (hereinafter referred to as P+ high fat diet). The composition of the high fat diet is shown in Table 1.

**[Table 1]**

| Composition of high fat diet (%) | |
|---|---|
| Sucrose | 50.00 |
| Milk casein | 20.00 |
| Cacao butter | 15.00 |
| Mineral mixed (AIN-76) | 5.00 |
| Cellulose | 4.95 |
| Cholesterol | 1.25 |
| Corn oil | 1.00 |
| Vitamin mixed (AIN-76) | 1.00 |
| Choline chloride | 1.00 |
| Sodium cholate | 0.50 |
| DL-methionine | 0.30 |
| Total | 100.00 |

The B+ high fat diet and the P+ high fat diet were prepared by removing cellulose in amounts corresponding to 0.60% and 0.50%, respectively, from the high fat diet of the above composition, and adding the corresponding amounts of BO-653 and probucol, respectively, to the remaining composition. Probucol is a liposoluble antioxidant. The inclusion herein of the study incorporating probucol into the high fat diet, as shown above, was designed to show that the therapeutic activity of BO-653 against fatty liver and associated hepatic disease is different from a protective action against hepatocyte damage which is caused by antioxidants hitherto considered to be effective for the treatment of hepatic disease.

At 37 weeks from the start of feeding to the respective groups, laparotomy was performed in each animal under ether anesthesia, and blood was collected from the inferior vena cava. Serum was separated from the collected blood, and AST and total cholesterol in the serum were measured with an autoanalyzer (COBS FARAII ROCHE). The entire body of the animal subjected to the laparotomy and blood collection was perfused with physiological saline, and the weight of the liver was measured. The results are shown in Table 2.

[Table 2]

**Table 2: Effect of BO-653 on excess apoAI expressing mice fed with high fat diet**

| Group | Ordinary diet | High fat diet | B+ high fat diet | P+ high fat diet |
|---|---|---|---|---|
| N | 5 | 7 | 7 | 8 |
| Liver weight (mg) | 1299±27** | 3163±121 | 2789±106* | 2923±185 |
| AST (U/L) | 36±1** | 242±26 | 96±7** | 218±18 |
| Total cholesterol (mg/dL) | 198±5** | 349±29 | 353±32 | 203±10** |

| | | | | |
|---|---|---|---|---|
| Mean ± standard error, *:p<0.05, **:P<0.01 | | | | |

As shown in Table 2, the weight of the liver tissue was greatly increased in the group fed with the high fat diet only, as compared with the group fed with the normal diet. Also, the appearance of the liver was yellow, clearly showing fatty liver. Furthermore, the blood AST level increased to about 7 times that in the normal diet group. Thus, it is clear that injury occurred in the liver cells according to the fatty liver, bringing about a large amount of AST leakage, namely, the onset of hepatic disease.

In the B+ high fat diet group, on the other hand, the liver weight was significantly less, and the blood AST was greatly lower, than in the high fat diet group. These findings demonstrate that BO-653 suppresses hepatomegaly due to fatty liver, and is effective for treatment of associated hepatic disease.

In the P+ high fat diet group, by contrast, the decreases in the liver weight and the blood AST were not as great as those in the B+ high fat diet group. It is seen that the protective action on liver cells by an antioxidant, such as probucol, is not sufficient for the treatment of fatty liver and associated hepatic disease. In connection with the decline in the blood AST, namely, the suppression of AST leakage from liver cells, in particular, the action of BO-653 is several times as high as that of probucol. Such a great difference suggests that the AST leakage suppressing action of BO-653 is mediated by a mechanism different from that of a cell protecting action by probucol.

The results in the B+ high fat diet group suggest that the AST leakage suppressing action of BO-653 is not affected by high blood total cholesterol levels, whereas the results in the P+ high fat diet group suggest that the decrease in the blood total cholesterol is not linked to the suppression of AST leakage. Both results suggest that there is no relationship between the AST leakage suppressing action and the blood total cholesterol.

The same tests were conducted using C57BL/6J mice (female, 6 weeks of age, purchased from CLEA Japan) expressing apolipoprotein AI normally, and apoAI deficiency mice (female, 10 to 11 weeks of age, purchased from Jackson Laboratories) expressing no apolipoprotein AI in contrast to the animals in the present Test Example. In both types of experimental animals, the blood ASL was significantly lower in the B+ high fat diet group than in the group receiving only the high fat diet (C57BL/6J mice, only high fat diet: 275±31, B+ high fat diet: 181±18; apoAI deficiency mice, only high fat diet: 159±10, B+ high fat diet: 85±8).

Besides, the effect of BO-653 was tested using apoE deficiency mice. These mice are known as mice showing high blood total cholesterol levels even on a normal diet and, at the same time, show high blood AST even on a normal diet. The results of the test were that BO-653 lowered only the blood AST without involving changes in blood total cholesterol values.

In summary, BO-653 has been shown to be useful for the prevention and treatment of fatty liver, and further for the prevention and treatment of hepatic disease associated with fatty liver. Its action is different from the cell protecting action of antioxidants such as probucol, and is obtained by its unique mechanism of directly suppressing the leakage of AST from liver cells. This mechanism has nothing to do with the level of blood total cholesterol.

The above Examples use BO-653 as a representative example of the compound of the formula (1), but the present invention is not limited to these Examples. A person skilled in the art will understand that other compounds of the formula (1) analogous to BO-653 have the same activity.

### INDUSTRIAL APPLICABILITY

According to the present invention, a pharmaceutical composition and a method for prevention and/or treatment of fatty liver or hepatic disease are provided.

## Claims

**1.** A pharmaceutical composition for prevention and/or treatment of fatty liver or hepatic disease, which comprises, as an active ingredient, a compound of the formula (1): 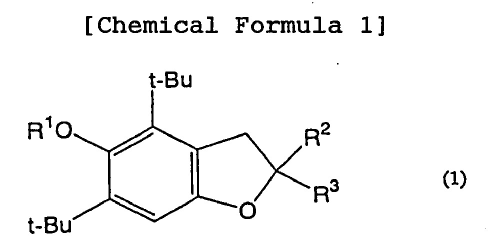 where
R¹ is a hydrogen atom, an acyl group, or an arylalkoxycarbonyl group; and
R² and R³ are each independently a substituted or unsubstituted alkyl group, a substituted or unsubstituted alkenyl group, or a substituted or unsubstituted alkynyl group, or R² and R³ may combine to form a cycloalkyl group.

**2.** The composition according to claim 1, wherein R¹ is a hydrogen atom.

**3.** The composition according to claim 1, wherein R² and R³ are each an unsubstituted alkyl group.

**4.** The composition according to claim 3, wherein the unsubstituted alkyl group is an n-butyl group, an n-pentyl group, an n-hexyl group, or an n-heptyl group.

**5.** The composition according to claim 1, wherein the compound of the formula (1) is 4,6-di-t-butyl-5-hydroxy-2,2-di-n-butyl-2,3-dihydrobenzofuran, 4,6-di-t-butyl-5-hydroxy-2,2-di-n-pentyl-2,3-dihydrobenzofuran, 4,6-di-t-butyl-5-hydroxy-2,2-di-n-hexyl-2,3-dihydrobenzofuran, or 4,6-di-t-butyl-5-hydroxy-2,2-di-n-heptyl-2,3-dihydrobenzofuran.

**6.** The composition according to claim 5, wherein the compound of the formula (1) is 4,6-di-t-butyl-5-hydroxy-2,2-di-n-pentyl-2,3-dihydrobenzofuran.

**7.** The composition according to claim 1, wherein the prevention and/or treatment of hepatic disease are or is ascribed to leakage of hepatic enzymes.

**8.** The composition according to claim 1, wherein the fatty liver is nonalcoholic fatty liver.

**9.** The composition according to claim 1, wherein the hepatic disease is hepatic disease associated with the fatty liver.

**10.** The composition according to claim 9, wherein the fatty liver is nonalcoholic fatty liver.

**11.** The composition according to claim 1, wherein the hepatic disease is bacterial or chemical-induced hepatic function disorder.

**12.** The composition according to claim 1, wherein the hepatic disease is chronic or acute hepatitis.

**13.** The composition according to claim 12, wherein the hepatitis is viral.

**14.** The composition according to claim 1, wherein the hepatic disease is hepatic cirrhosis.

**15.** The composition according to claim 1, wherein the hepatic disease is liver cancer.

**16.** Use of a compound of the formula (1): 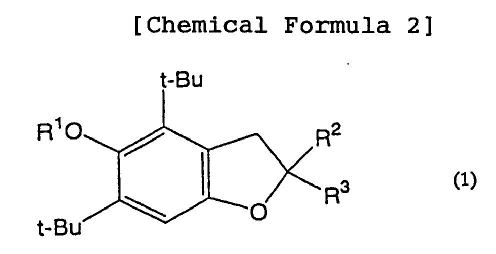 where
R¹ is a hydrogen atom, an acyl group, or an arylalkoxycarbonyl group; and
R² and R³ are each independently a substituted or unsubstituted alkyl group, a substituted or unsubstituted alkenyl group, or a substituted or unsubstituted alkynyl group, or R² and R³ may jointly form a cycloalkyl group, in production of a pharmaceutical composition for prevention and/or treatment of fatty liver or hepatic disease.

**17.** A method for prevention and/or treatment of fatty liver or hepatic disease, comprising administering a compound of the formula (1): 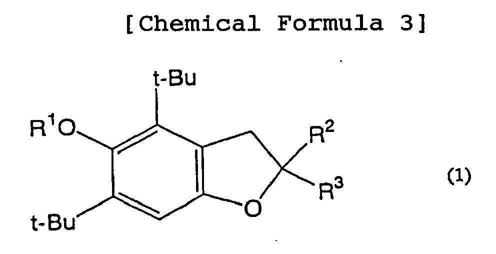 where
R¹ is a hydrogen atom, an acyl group, or an arylalkoxycarbonyl group; and
R² and R³ are each independently a substituted or unsubstituted alkyl group, a substituted or unsubstituted alkenyl group, or a substituted or unsubstituted alkynyl group, or R² and R³ may jointly form a cycloalkyl group, to a patient in need of such prevention and/or treatment.
